# EUROPÄISCHE PATENTSCHRIFT

(11) **EP 2 937 692 B1**
(45) Veröffentlichungstag und Bekanntmachung des Hinweises auf die Patenterteilung: **07.06.2017**
(21) Anmeldenummer: 15162215.6
(22) Anmeldetag: 01.04.2015
(51) Int. Cl.: G01N 33/03, G01N 27/22, A47J 37/12

(54) **ÖLQUALITÄTSSENSOR UND FRITTIERVORRICHTUNG MIT EINEM SOLCHEN ÖLQUALITÄTSSENSOR**
OIL QUALITY SENSOR AND DEEP FRYING DEVICE WITH SUCH AN OIL QUALITY SENSOR
CAPTEUR DE QUALITE D'HUILE ET APPAREIL DE FRITURE DOTE D'UN TEL CAPTEUR DE QUALITE D'HUILE

(30) Priorität: 04.04.2014 DE 102014104843
(43) Veröffentlichungstag der Anmeldung: 28.10.2015
(73) Patentinhaber: WTW Wissenschaftlich-Technische Werkstätten GmbH, 82362 Weilheim (DE)
(72) Erfinder: Baumann, Michael, 86633 Neuburg (DE); Beyer, Klaus, 85055 Ingolstadt (DE); Ottenthal, Thomas, 86316 Friedberg (DE)
(74) Vertreter: Schlief, Thomas P.

(56) Entgegenhaltungen:
- CH-A5- 692 826
- CN-U- 202 837 286
- DE-C1- 19 511 556
- DE-U1-202005 007 144
- US-A1- 2009 193 873
- US-A1- 2012 182 030

## Beschreibung

Die Erfindung betrifft einen Ölqualitätssensor zur Ermittlung der Qualität von Frittieröl durch Messung der Kapazität des Frittieröls in einer Frittiereinrichtung.

Es sind Vorrichtungen zur Messung der Frittierölqualität bekannt, bei denen eine Frittierölprobe dem Frittierbecken entnommen und die Frittierölqualität außerhalb der Frittiereinrichtung in einer Messvorrichtung mittels eines Kondensators gemessen wird. Diese Vorrichtung macht es sich zunutze, dass die Dielektrizitätskonstante des Frittieröls sich mit seiner Alterung verändert. Über eine Korrelationsfunktion können aus der Änderung der Dielektrizitätskonstanten die polaren Anteile des Frittieröls berechnet werden.

Des Weiteren ist es beispielsweise aus der DE 10015516 bekannt, die Qualität von Frittieröl mittels einer in einer Hand haltbaren Vorrichtung zu messen. Zur Durchführung der Messung ergreift der Bediener das Gehäuse, in dem die Messelektronik angeordnet ist. Auf der Unterseite des Gehäuses ist ein Metallrohr befestigt, an dessen freiem Ende ein frei liegender InterdigitalKondensator angeordnet ist. Der Kondensator ist mittels in dem Metallrohr verlaufender elektrischer Leitungen mit der Messelektronik verbunden, wobei ein Teil dieser Elektronik auch in dem Metallrohr untergebracht ist. Neben der Kapazität (und damit der Dielektrizitätskonstanten) wird mittels der bekannten Vorrichtung außerdem die Temperatur des Frittieröls gemessen, da die Dielektrizitätskonstante nicht nur von der Frittierölqualität, d.h. den polaren Anteilen, sondern auch stark von der Temperatur des Frittieröls abhängig ist. Die gemessene Öltemperatur geht direkt in die Berechnung der polaren Anteile ein. Demnach kann aus den Messwerten zur Kapazität und zur Temperatur auf die Menge der polaren Anteile im Frittieröl und damit der Frittierölqualität geschlossen werden.

Eine ähnliche, ebenfalls in der Hand zu haltende Messvorrichtung ist auch aus der DE 20 2005 007 144 U1 bekannt. Bei dieser ordnet eine Auswerteelektronik bei Messung in mindestens einem ersten Prüföl, welches nominell keine polaren Anteile enthält und eine bekannte Dielektrizitätskonstante besitzt, die gemessene Dielektrizitätskonstante einem fiktiven Gehalt an polaren Anteilen zu. Wenn der Messwert der Dielektrizitätskonstanten bzw. der zugeordnete fiktive Gehalt an polaren Anteilen innerhalb bzw. außerhalb vorgegebener oder vom Benutzer zu wählender Grenzbereiche liegt, wird dem Benutzer mittels der Ausgabeeinheit signalisiert, dass die Messvorrichtung bestimmungsgemäß einsetzbar bzw. neu zu justieren ist.

Des Weiteren ist aus der US 8,497,691 B1 bekannt, einen stationären Ölqualitätssensor in einem Filtrierkreislauf, in einem Rücklauf oder in einer Frittierwanne anzuordnen. Der Ölqualitätssensor weist - wie das oben genannte Handmessgerät - einen Interdigitalkondensator auf.

In der US 2009/0193873 A1 ist ein Benzinqualitätssensor offenbart, der eine erste zylinderförmige Elektrode mit einem ersten Durchmesser und eine zweite zylinderförmige Elektrode mit einem kleineren als dem ersten Durchmesser offenbart, wobei die erste Elektrode die zweite Elektrode umschließt und beide Elektroden koaxial zueinander angeordnet sind. Dieser Sensor ist parallel zur Strömungsrichtung von Benzin in einem Rohr angeordnet, wobei das Benzin zwischen den beiden Elektroden hindurch fließt. Zudem ist ein Temperatursensor im Strömungsweg angeordnet. Mit Hilfe einer entsprechend eingerichteten Auswerteeinheit lässt sich durch Messung der Kapazität zwischen diesen beiden Elektroden sowie der Temperatur die Qualität des Benzins ermitteln.

Nachteilig bei den oben zuerst genannten, tragbaren Vorrichtungen ist, dass sie stets das Aktivwerden des Bedieners erfordern. Die Messaufgabe kann im hektischen Betrieb eines Schnellrestaurants jedoch häufig vergessen werden. Auch muss mit der tragbaren Vorrichtung relativ sorgsam umgegangen werden, da ansonsten der Kondensator bei Berührung mit beispielsweise dem Frittierbecken Schaden nehmen kann. Der bekannte stationäre Ölqualitätssensor und der bekannte stationäre Benzinqualitätssensor hingegen werden hinsichtlich ihrer Messpräzision nicht den allerhöchsten Anforderungen gerecht.

Es ist Aufgabe der vorliegenden Erfindung, einen Ölqualitätssensor zur Verfügung zu stellen, der eine einfachere und sicherere Handhabung einerseits und eine hohe Messpräzision andererseits gewährleistet.

Diese Aufgabe wird durch einen Ölqualitätssensor gemäß dem Anspruch 1 gelöst.

Die Vorteile der Erfindung sind insbesondere darin zu sehen, dass der Ölqualitätssensor, der permanent in der Frittiereinrichtung installiert werden kann, einen Kondensator aufweist, zwischen dessen beiden Elektroden das Frittieröl hindurchgeführt wird. Der Ölqualitätssensor kann also als Durchflusssensor oder bei stehendem Frittieröl betrieben werden. Durch das Hindurchführen des Frittieröls durch einen langgestreckten Kondensator wird die Messpräzision gegenüber einem Interdigitalkondensator deutlich erhöht.

Wird der erfindungsgemäße Ölqualitätssensor von Frittieröl durchspült, hat das Frittieröl in den meisten Fällen eine andere Temperatur als der Ölqualitätssensor selbst. Da das Frittieröl aus einem heißen Frittierbecken in den Ölqualitätssensor gelangt, ist es üblicherweise deutlich wärmer als der Ölqualitätssensor selbst. Aus diesem Grund wird sich bei einem Zylinderkondensator ein Temperaturgradient zwischen der Außenseite des Ölqualitätssensors und der Innenelektrode bilden, wobei die Temperatur des Frittieröls zwischen der äußeren und der inneren Elektrode nicht homogen ist. Aufgrund dieses Temperaturgradienten ist erfindungsgemäß ein zweiter Temperatursensor vorgesehen, der an einer anderen Stelle als der erste Temperatursensor platziert wird. Vorteilhafterweise ist einer der Temperatursensoren nahe oder an der ersten Elektrode und der andere Temperatursensor nahe oder an der zweiten Elektrode platziert. Besonders bevorzugt wird aus den von den beiden Temperatursensoren ermittelten Messwerten von der Auswerteeinheit eine kombinierte Temperatur berechnet, vorzugsweise eine mittlere, effektive Temperatur, welche dann von der Auswerteeinheit für die Ermittlung der polaren Anteile verwendet wird.

Gemäß einer besonders bevorzugten Ausführungsform ist der Kondensator als Zylinderkondensator ausgebildet. Hierbei umgibt die zweite (äußere) Elektrode die erste (innere) Elektrode unter Ausbildung eines Zwischenraums, durch den das Frittieröl (als Dielektrikum des Kondensators) hindurchleitbar ist. In diesem Fall ist eine Integration des Ölqualitätssensors in den bestehenden Ölkreislauf besonders einfach möglich. Zudem kann durch die längliche Ausgestaltung des Kondensators als Zylinderkondensator eine große Messfläche realisiert werden, so dass sehr präzise Messungen möglich sind. Auch ist es möglich, den Leitungsquerschnitt des Zylinderkondensators an die übrigen Leitungen der Frittiervorrichtung anzupassen, so dass der erfindungsgemäße Ölqualitätssensor keine Druckänderungen, Verwirbelungen und andere unerwünschte Effekte hervorruft.

Eine ebenfalls vorteilhafte Alternative sieht vor, dass der Kondensator als Rohrkondensator ausgebildet ist, bei dem die beiden Elektroden als vorzugweise im Wesentlichen gleich große Halbschalen ausgebildet sind, die zusammen im Querschnitt einen fast geschlossenen Kreis bilden. Die konkaven Flächen dieser beiden Elektroden liegen sich unter Ausbildung eines Zwischenraums gegenüber, wobei auch hier das Frittieröl durch diesen Zwischenraum hindurchgeleitet wird. Die Vorteile eines Rohrkondensators sind mit denjenigen eines Zylinderkondensators vergleichbar.

Eine vorteilhafte Ausgestaltung in Bezug auf den zweiten Temperatursensor sieht vor, dass - bei Ausbildung des Kondensators als Zylinderkondensator - der eine Temperatursensor innerhalb eines Hohlabschnitts der ersten, inneren Elektrode und/oder der andere Temperatursensor auf der der ersten Elektrode abgewandten Außenseite der zweiten, äußeren Elektrode angeordnet ist. Hierdurch kann dem Temperaturgradienten - bei gleichzeitiger geschützter Anbringung der Temperatursensoren außerhalb des Durchflussbereichs für das Frittieröl - in vorteilhafter Weise Rechnung getragen werden. Es ist aber auch selbstverständlich möglich, einen oder beide Temperatursensoren im Durchflussbereich des Frittieröls anzuordnen.

Es ist im Übrigen nicht ausgeschlossen, dass zur weiteren Steigerung der Berechnungsgenauigkeit der polaren Anteile noch ein dritter (oder noch weitere) Temperatursensor(en) an unterschiedlichen Stellen im Ölqualitätssensor platziert werden.

Es sei an dieser Stelle angemerkt, dass die Auswerteeinheit ihre Aufgabe auch über verschiedene elektronische Bauteile verteilt durchführen kann, dass also die Auswerteeinheit beispielsweise nicht in lediglich einem einzigen Gehäuse oder auf einer einzigen Platine untergebracht sein muss.

Gemäß einer zweckmäßigen Ausgestaltung ist um die Elektroden herum eine Schirmelektrode vorgesehen, welche Störsignale von außen abschirmt. Die Messgenauigkeit und Reproduzierbarkeit kann hierdurch wesentlich gesteigert werden.

Es hat sich als besonders vorteilhaft erwiesen, wenn die Mittelachse der Zulauf- und/oder Ablauföffnung in einem Winkel, vorzugsweise in einem Winkel von 90°, zur Längsachse des Kondensators bzw. des Hohlraums verläuft, d.h. unter einem Winkel größer als 0° in den Hohlraum mündet. Wenn beide, die Zulauf- und die Ablauföffnung, in Axialrichtung des Kondensators verlaufen würden, wäre ein Anschluss der elektrischen Leitungen für die beiden Elektroden und für den bzw. die Temperatursensoren nur relativ aufwändig zu realisieren. Durch die Abwinklung von mindestens der Mittelachse der Zulauföffnung oder der Ablauföffnung in Bezug auf die Längsachse des Kondensators können die elektrischen Leitungen von der Seite der abgewinkelten Zulauf- bzw. Ablauföffnung zugeführt werden. Hierbei verlaufen diese elektrischen Leitungen vorzugsweise im Wesentlichen in Axialrichtung des Kondensators bzw. des Hohlraums, also in dessen rückwärtiger Verlängerung, welche aufgrund der besagten Abwinklung für eine derartige Verlegung der elektrischen Leitungen nicht obstruiert ist.

Dementsprechend schließt sich bevorzugt an einer Stirnseite des Hohlraums rückwärtig eine Kammer an, welche zur Aufnahme der Messelektronik, insbesondere der Auswerteeinheit, dient. Die Messelektronik ist hierbei mit den zu den beiden Elektroden und dem bzw. den Temperatursensoren führenden elektrischen Leitungen verbunden. Vorzugsweise ist des Weiteren eine digitale Schnittstelle an oder in der Kammer angeordnet, welche mit der Messelektronik verbunden ist. Über die Schnittstelle können Signale, insbesondere die Messwerte und/oder die berechneten Werte zu den polaren Anteilen und/oder ein die Ölqualität angebendes Signal (z.B. "grün" für "Ölqualität in Ordnung" und "rot" für "Frittieröl muss gewechselt werden") ausgegeben werden. Auch kann die Messelektronik über die Schnittstelle programmiert werden, beispielsweise der Messzyklus eingestellt werden.

Bei der genannten Ausgestaltung einer abgewinkelten Mittelachse der Zulauf- oder der Ablauföffnung verläuft die Mittelachse der anderen Öffnung, also die Mittelachse der Ablauf- oder der Zulauföffnung, vorzugsweise parallel und besonders bevorzugt koaxial zur Längsachse des Kondensators. Hierdurch ergibt sich ein kompakter Aufbau mit hervorragender Raumausnutzung, Durchspülung des Ölqualitätssensors sowie Anschlussmöglichkeiten, sowohl hinsichtlich der das Frittieröl führenden Durchflussleitungen als auch der elektrischen Leitungen.

Entsprechend dem Vorgesagten verläuft bei einer diesbezüglichen konkreten und vorteilhaften Ausgestaltung des Ölqualitätssensors die Mittelachse der Zulauföffnung in einem Winkel, vorzugsweise in einem Winkel von 90°, zur Längsachse des Kondensators, während die Mittelachse der Ablauföffnung parallel und vorzugweise koaxial zu der Längsachse des Kondensators verläuft. Hierbei sind die zu den beiden Elektroden und dem oder den Temperatursensoren führenden elektrischen Leitungen von der Seite der abgewinkelten Zulauföffnung des Ölqualitätssensors her zugeführt, vorzugsweise im Wesentlichen in Axialrichtung des Hohlraums des Ölqualitätssensors.

Zweckmäßigerweise weist der erfindungsgemäße Ölqualitätssensor einen elektronischen Speicher auf, in dem Korrelationsfunktionen hinterlegt sind. Diese geben die Korrelation zwischen den Kapazitätsmesswerten und unterschiedlichen Ölsorten wider, welche - auch auf internationaler Ebene - für das Frittieren von Frittiergut (z.B. Pommes Frites, Hühnchenstücke, usw.) Verwendung finden. Auf diese Korrelationsfunktionen mit den entsprechend hinterlegten Korrelationswerten greift die Auswerteeinheit je nach ausgewählter Ölsorte zur Berechnung der polaren Anteile unter Verwendung der gemessenen Kapazität und der Temperatur zu. Es lassen sich somit präzise Qualitätsurteile für eine Vielzahl von unterschiedlichen Frittierölsorten erhalten. Der erfindungsgemäße Ölqualitätssensor ist demnach flexibel und universal einsetzbar.

Die Erfindung umfasst ebenfalls eine Frittiervorrichtung nach Anspruch 10, in welche demnach ein wie vorbeschrieben erfindungsgemäßer Ölqualitätssensor integriert ist. Diese Frittiervorrichtung ist leicht zu bedienen, der Ölqualitätssensor durch die Integration geschützt und aufgrund seiner Ausgestaltung für eine sichere, einfache und sehr präzise Qualitätsbestimmung des Frittieröls hervorragend geeignet.

Für den Einbau des Ölqualitätssensors in die Filtereinrichtung kommen unterschiedliche Stellen in Frage. Beispielsweise kann der Ölqualitätssensor in einen Ölkreislauf integriert sein, in welchem auch die Filtereinrichtung platziert ist. Es bietet sich hierbei an, den Ölqualitätssensor nach der Filtereinrichtung anzuordnen, damit sich keine Frittierreste usw. im Ölqualitätssensor ablagern können. Bei einer Alternative ist ein Nebenkreislauf (Messkreislauf) vorgesehen, in welchen der Ölqualitätssensor eingebaut ist.

Einem gleichmäßigen Durchfluss für das Frittieröl - ohne die Erzeugung von Verwirbelungen etc. - kommt es vorteilhafterweise zugute, wenn der Ölqualitätssensor einen Durchflussquerschnitt aufweist, der nicht mehr als 25% vom Durchflussquerschnitt der zum Ölqualitätssensor hin- und vom Ölqualitätssensor wegführenden Leitungen abweicht. Bevorzugt sind die Durchflussquerschnitte im Wesentlichen gleich groß.

Besonders bevorzugt ist der Ölqualitätssensor derart in der Frittiervorrichtung angeordnet, dass die Längsachse des Kondensators einen Winkel im Bereich von 20° bis 90°, vorzugsweise größer als 30°, mit der Horizontalen bildet, wobei die Ablauföffnung bevorzugt nach oben weist. Hierdurch lässt sich die Präzision der Messungen wie folgt steigern: Sobald der Sensor mit Öl gefüllt ist, kann über die Messung der Kapazität und der Temperatur die Ölqualität bestimmt werden. Geschieht dies, während Frittieröl durch den Sensor gepumpt wird, können ggf. im Ölfluss enthaltene Luftblasen sowie Wasser das Messergebnis stören. Wird der Sensor durch eine Pumpe gefüllt, der Ölfluss gestoppt und eine gewisse Zeit gewartet, kann im Frittieröl enthaltene Luft, Wasserdampf und/oder Wasser den Zwischenraum zwischen den beiden Elektroden verlassen. Das Messergebnis wird dann nur noch durch das Frittieröl bestimmt. Luft und Wasserdampf können den besagten Zwischenraum beispielsweise verlassen, indem diese gasförmigen Verbindungen aus der Ablauföffnung des Ölqualitätssensors nach oben entweichen. Dies wird durch eine Montage des Ölqualitätssensors in einem Winkel zwischen 20° und 90°, vorzugsweise größer als 30° begünstigt. Wasser kann sich unterhalb des besagten Zwischenraumes zwischen den beiden Elektroden im Ölqualitätssensor absetzen.

Auch umfasst die Erfindung ein entsprechendes diskontinuierliches Messverfahren nach Anspruch 15, wobei nach Füllen des Ölqualitätssensors mit Frittieröl gewartet wird, bis Luft, Wasserdampf und/oder Wasser den Zwischenraum zwischen den Elektroden bzw. den Ölqualitätssensor verlassen haben, um dann die Kapazitätsmessung durchzuführen. Die schräge Anordnung des Ölqualitätssensors, wie zuvor beschrieben, ist hierbei bevorzugt.

Mittels des erfindungsgemäßen Ölqualitätssensors bzw. der erfindungsgemäßen Frittiervorrichtung lässt sich vorteilhafterweise die Anwesenheit von Luft im Filterkreislauf erfassen. Befindet sich Luft im Filterkreislauf, so führt dies zu einer deutlich schnelleren Alterung des Frittieröls, da die Oxidation des Öls stark erhöht wird. Luft im Filterkreislauf ist daher möglichst zu vermeiden bzw. zu minimieren. Um abschätzen zu können, ob (zu) viel Luft im Ölkreislauf vorhanden ist, wird während des Pumpvorgangs beim Filtern eine kontinuierliche Kapazitätsmessung durchgeführt. Somit können Luftblasen anhand der Signaländerungen detektiert werden. Je mehr Luft im System ist, desto größer werden die Messsignalschwankungen während des Pumpvorgangs. Die Auswerteeinheit detektiert und wertet diese Signalschwankungen aus, um sie vorteilhafterweise dann als Maß für die Luftmenge an ein übergeordnetes System, z.B. die Steuerung der Frittiervorrichtung, weiterzuleiten. Die Steuerung kann mit dieser Information dem Anwender z.B. mitteilen, den Service zu informieren, damit dieser die Ursache der Luftansammlung im System beheben kann.

Auch als Füllstandsensor lässt sich der erfindungsgemäße Ölqualitätssensor verwenden, da der Signalunterschied zwischen einem gefüllten und einem ungefüllten Sensor sehr deutlich ist. Dies kann z.B. dafür genutzt werden, die Pumpe einer Filtereinrichtung zu stoppen, sobald sich kein Frittieröl mehr in der Filterwanne befindet, d.h. das gesamte Öl in das Frittierbecken gepumpt wurde. Wenn demnach ein Unter- oder Überschreiten eines Schwellwerts der gemessenen Kapazität über einen vorzugsweise vorgegebenen Zeitraum hinweg detektiert wird, kann dies beispielsweise an die Steuereinrichtung der Frittiereinrichtung übermittelt werden. Diese kann dann aus der Schwellwertunter- oder -überschreitung auf einen unterbrochenen Ölfluss durch den Ölqualitätssensor schließen und die Ausgabe eines entsprechenden Signals veranlassen, beispielsweise eines Warnsignals oder eines Steuersignals zur Abschaltung der Pumpe.

Vorteilhafte Weiterbildungen der Erfindung sind durch die Merkmale der Unteransprüche gekennzeichnet. Im Folgenden wird die Erfindung anhand von Figuren näher erläutert, wobei gleiche Bezugszeichen für gleiche oder gleichwertige Elemente Verwendung finden. Es zeigen:
- **Figur 1**: einen erfindungsgemäßen Ölqualitätssensor im Längsschnitt;
- **Figur 1a**: einen Querschnitt durch den Ölqualitätssensor der Figur 1 längs A-A;
- **Figur 1b**: einen Querschnitt durch eine alternative Ausführungsform eines Ölqualitätssensors;
- **Figur 2**: eine schematische Prinzipskizze zu einer ersten möglichen Anordnung des Ölqualitätssensors in einer Frittiervorrichtung, und
- **Figur 3**: eine schematische Prinzipskizze zu einer zweiten möglichen Anordnung des Ölqualitätssensors in einer Frittiervorrichtung.

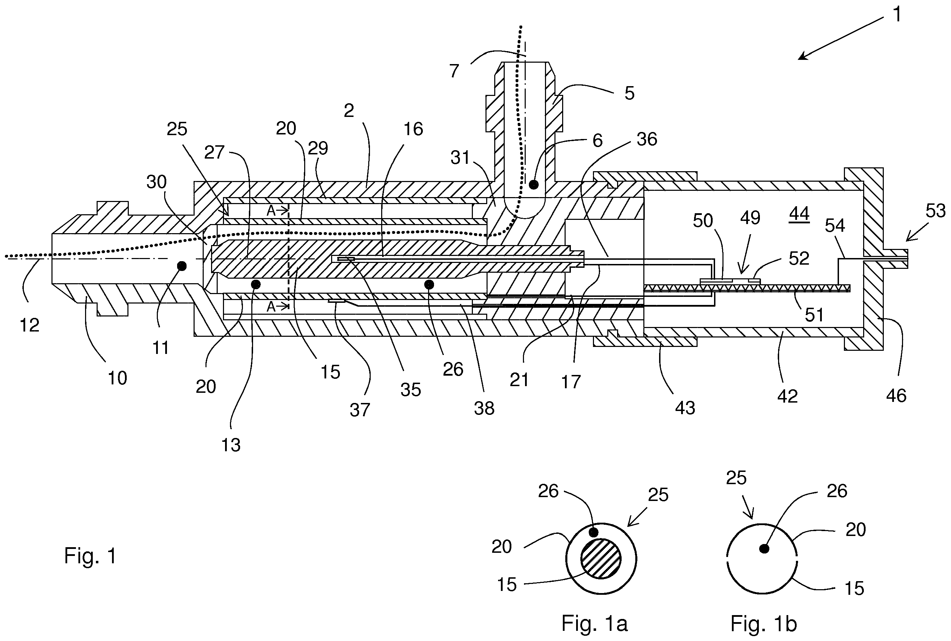

In der Figur 1 ist ein erfindungsgemäßer Ölqualitätssensor 1 im Längsschnitt dargestellt.. Dieser weist ein Gehäuse 2 auf, in dem ein Hohlraum 13 vorgesehen ist. An dem Hohlraum 13 ist rechtwinklig ein Flansch 5 angebracht, der eine Zulauföffnung 6 zum Einleiten von Frittieröl aufweist. In Verlängerung des Gehäuses 2 ist andererseits eine Ablauföffnung 11 an einem weiteren Flansch 10 angeordnet, durch welche das Frittieröl den Hohlraum 13 verlassen kann. Die Zulauf- und die Ablauföffnungen 6, 11, die vorzugsweise einen runden Querschnitt besitzen, weisen jeweils eine Mittelachse 7 bzw. 12 auf, wobei diese beiden Achsen 7, 12 vorliegend senkrecht zueinander verlaufen.

In dem Hohlraum 13 ist entlang seiner Längsachse 27 eine längliche erste Elektrode 15 angeordnet, die vorder- und rückseitig in einem vorderen bzw. einem hinteren Zentrierelement 30, 31 gelagert ist. In Umfangsrichtung herum ist um die erste Elektrode 15 eine zweite, sich ebenfalls in Längsrichtung des Hohlraums 13 erstreckende Elektrode 20 angeordnet, so dass sich die Außenseite der ersten Elektrode 15 und die Innenseite der zweiten Elektrode 20 beabstandet gegenüber liegen. Sowohl die erste als auch die zweite Elektrode 15, 20 sind im Querschnitt kreisförmig ausgebildet und konzentrisch zueinander angeordnet, s. die Querschnittsdarstellung in Fig. 1a. Sie bilden zusammen einen Kondensator 25 mit einem zwischen den Elektroden 15, 20 liegenden Zwischenraum 26, der Teil des Hohlraums 13 ist. Frittieröl, das durch die Zulauföffnung 6 in den Ölqualitätssensor 1 einfließt, strömt dann durch den Zwischenraum 26 in Richtung der Ablauföffnung 11 (angedeutet durch die punktierte Linie, hier dargestellt als Fluss entlang eines oberen Durchflusspfades).

Die beiden Elektroden 15, 20 umgibt eine schalenförmige, im Querschnitt kreisrunde und ebenfalls entlang des Hohlraums 13 verlaufende, lang gestreckte, metallische und gegenüber dem Gehäuse 2 elektrisch isolierte Schirmelektrode 29, welche konzentrisch zu den besagten Elektroden 15, 20 angeordnet ist und an der Innenseite des Gehäuses 2 anliegt. Die Schirmelektrode 29, die beabstandet zur zweiten Elektrode 20 angeordnet ist, dient zur Abschirmung gegenüber von außen eindringenden Störsignalen. Durch die Isolierung gegenüber dem Gehäuse 2 kann für die Schirmung ein anderes elektrisches Potential verwendet werden als die elektrische Masse des Gehäuses 2. Das ist beispielsweise vorteilhaft, wenn das Gehäuse 2 auf Erdpotential liegt (was beim Einbau in Frittiervorrichtungen regelmäßig der Fall ist), wodurch unter Umständen Störungen von der Erde auf die Gehäusemasse übertragen werden.

Ein erster Temperatursensor 35, beispielsweise ein PT1000, ist in einen Hohlabschnitt 16 der ersten Elektrode 15 eingeschoben, wobei der Hohlabschnitt von einer entlang der Längsachse 27 des Kondensators (die mit der Längsachse 27 des Hohlraums 13 zusammen fällt) verlaufenden Sackbohrung gebildet ist.

Ein zweiter Temperatursensor 37, ebenfalls beispielsweise ein PT1000, ist an der Außenseite der zweiten Elektrode 20 vorgesehen, welcher die dortige Temperatur misst.

Die beiden Temperatursensoren 35, 37, die ungefähr jeweils in der Mitte der beiden Elektroden 15, 20 angeordnet sind, liefern jeweils Temperaturmesswerte, aus welchen eine mittlere Temperatur ermittelt wird, welche ein genaueres Abbild der im Ölqualitätssensor 1 herrschenden Temperaturen liefert. Insbesondere kann einem zwischen der ersten und der zweiten Elektrode 15, 20 herrschenden Temperaturgradienten Rechnung getragen werden. Da die Temperatur direkt in die Berechnung der polaren Anteile - zusammen mit der gemessenen Kapazität des Kondensators 25, bei dem das Frittieröl das Dielektrikum bildet - eingeht, ist eine möglichst präzise Ermittlung einer effektiven Temperatur erwünscht.

Die beiden Elektroden 15, 20 sind über elektrische Leitungen 17 bzw. 21 mit einer Messelektronik 49 verbunden, die auf einer Platine 51 angeordnet ist. Die Platine 51 ist in einer von einem rückwärtigen Gehäuse 42 gebildeten Kammer 44 befestigt, welche sich am der Ablauföffnung 11 gegenüber liegenden Ende des Hohlraums 13 befindet und mit einem ringförmigen Adapter 43 mit dem Gehäuse 2 verbunden ist. Auch die beiden Temperatursensoren 35, 37 sind über elektrische Leitungen 36 bzw. 38 mit der Messelektronik 49 verbunden. Zur Führung der elektrischen Leitungen 17, 21, 36, 38 zur Messelektronik 49 sind entsprechende Durchbrüche in dem hinteren Zentrierelement 31 vorgesehen.

Zur Erfassung der gemessenen Kapazitäten sowie der gemessenen Temperaturen, zur Digitalisierung der Messwerte und zur Berechnung der polaren Anteile ist eine Auswerteeinheit 50, vorliegend als Teil der Messelektronik 49, vorgesehen. Auf der Platine 51 - entweder als separates Element oder als Teil der Messelektronik 49 - ist des Weiteren ein elektronisches Speicherelement 52 angeordnet, in welchem Korrelationsfunktionen abgespeichert sind, welche den Zusammenhang zwischen den Kapazitätswerten und den unterschiedlichen Frittierölsorten wiedergeben. Diese Ausgestaltung erhöht die Messgenauigkeit, da jedes Frittierröl eine unterschiedliche Dielektrizitätskonstante besitzt.

Die Kammer 44 wird rückwärtig von einer Abdeckkappe 46 abgeschlossen, in der eine digitale Schnittstelle 53, beispielsweise in Form eines USB-Anschlusses, zum Ausgeben von Signalen der Auswerteeinheit 50, zum Abspeichern von neuen Korrelationsfunktionen, zum Programmieren von Messzyklen der Messelektronik 49 o.ä., vorgesehen ist.

Wie oben erwähnt, ist in der Figur 1 dargestellt, dass die Mittelachse 7 der Zulauföffnung 6 im rechten Winkel in den Hohlraum 13 mündet. Hierdurch ergibt sich die Möglichkeit eines einfachen Anschlusses der elektrischen Leitungen 17, 21, 36, 38, da diese nun - wie oben geschildert - im Wesentlichen linear in die rückwärtige Kammer 44 geführt werden können. Wären die Mittelachsen 7, 12 hingegen parallel zueinander bzw. sogar koaxial, würde sich die Führung der besagten elektrischen Leitungen 17, 21, 36, 38 sowie die Unterbringung der Messelektronik 49 wesentlich schwieriger gestalten.

In einer schematisch in Fig. 1b im Querschnitt (vgl. Fig. 1a) dargestellten alternativen Ausführungsform ist der Kondensator 25 als Rohrkondensator ausgebildet, bei dem die beiden Elektroden 15, 20 als Halbschalen ausgebildet sind, deren konkave Flächen sich unter Ausbildung eines Zwischenraums 26 gegenüber liegen, durch den das Frittieröl hindurchleitbar ist.

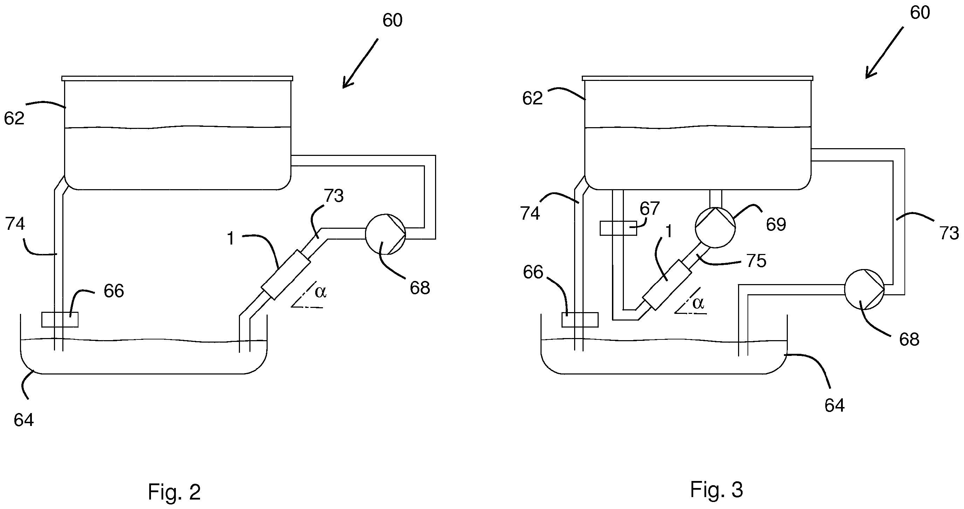

In den Figuren 2 und 3 sind sehr schematisch zwei Ausführungsformen einer Frittiervorrichtung 60 dargestellt, in denen ein Frittierbecken 62 zum Frittieren von Frittiergut über Zuführ- und Abführleitungen 73, 74 mit einer Frittierwanne 64 verbunden ist. Eine Filtereinrichtung 66 zum Filtern des Frittieröls zum Zwecke des Abscheidens von Frittierresten und anderen Verschmutzungen ist vorliegend in die Abführleitung 74 eingebaut. In der Zuführleitung 73 ist eine Pumpe 68 integriert, welcher der erfindungsgemäße Ölqualitätssensor 1 vorgeschaltet ist. Dieser ist in einem Winkel α von vorliegend ca. 45° zur Horizontalen angeordnet, damit - nach Schließen eines Ventils (nicht dargestellt) oder Abschalten der Pumpe und Abwarten eines kurzen Zeitraums zur Beruhigung des Öls in dem Ölqualitätssensor 1 - Luft und Wasserdampf nach oben aus der Ablauföffnung 11 entweichen und Wasser sich im unteren Teil des Ölqualitätssensors 1 unterhalb des Zwischenraums 26 absetzen kann.

In der Figur 3 ist der Ölqualitätssensor 1 in eine Leitung 75 eines Nebenkreislaufs (d.h.außerhalb des Frittierkreislaufs) eingebaut, wobei der Nebenkreislauf auch als Messkreislauf bezeichnet werden kann. In Nebenkreislauf ist ebenfalls eine Filtereinrichtung 67 und eine Pumpe 69 vorhanden.

Die Leitungen 73, 75, welche zum Ölqualitätssensor hin und weg führen, weisen bevorzugt im Wesentlichen den gleichen Durchflussquerschnitt auf wie dessen Zu- und Ablauföffnung 6, 11, um insbesondere Verwirbelungen oder andere Flussstörungen zu vermeiden. Bevorzugt sind Abweichungen der Querschnitte von weniger als 25%.

Mit dem Ölqualitätssensor lassen sich nicht nur die polaren Anteile im Frittieröl und damit dessen Qualität messen, sondern - wie oben beschrieben - auch die Anwesenheit von Luft und der Füllstand der Frittierwanne 64 (die hierzu eingesetzte Steuereinrichtung der Frittiervorrichtung 60 ist in den Figuren nicht dargestellt).

### Bezugszeichenliste

- 1: Ölqualitätssensor
- 2: Gehäuse
- 5: Flansch
- 6: Zulauföffnung
- 7: Mittelachse der Zulauföffnung
- 10: Flansch
- 11: Ablauföffnung
- 12: Mittelachse der Ablauföffnung
- 13: Hohlraum
- 15: Erste Elektrode
- 16: Hohlabschnitt
- 17: Leitung der ersten Elektrode
- 20: Zweite Elektrode
- 21: Leitung der zweiten Elektrode
- 25: Kondensator
- 26: Zwischenraum
- 27: Längsachse des Kondensators bzw. des Hohlraums
- 29: Schirmelektrode
- 30: Vorderes Zentrierelement
- 31: Hinteres Zentrierelement
- 35: Erster Temperatursensor
- 36: Leitung des ersten Temperatursensors
- 37: Zweiter Temperatursensor
- 38: Leitung des zweiten Temperatursensors
- 42: Rückwärtiges Gehäuse
- 43: Adapter
- 44: Kammer
- 46: Abdeckkappe
- 49: Messelektronik
- 50: Auswerteeinheit
- 51: Platine
- 52: elektronischer Speicher
- 53: Schnittstelle
- 60: Frittiervorrichtung
- 62: Frittierbecken
- 64: Frittierwanne
- 66: Filtereinrichtung
- 67: Filtereinrichtung
- 68: Pumpe
- 69: Pumpe
- 73: Zuführleitung
- 74: Abführleitung
- 75: Leitung

## Patentansprüche

1. Ölqualitätssensor zur Ermittlung der Qualität von Frittieröl durch Messung der Kapazität des Frittieröls in einer Frittiervorrichtung:
- mit einem Gehäuse (2) und einem Hohlraum (13) in dem Gehäuse (2), durch welchen das Frittieröl hindurchleitbar ist,
- mit einer Zulauföffnung (6) zum Einleiten von Frittieröl in den Hohlraum (13),
- mit einer Ablauföffnung (11) zum Ausleiten des Frittieröls aus dem Hohlraum (13),
- mit einer ersten sich entlang des Hohlraums (13) erstreckenden Elektrode (15),
- mit einer zweiten sich entlang des Hohlraums (13) erstreckenden Elektrode (20), die der ersten Elektrode (15) gegenüber angeordnet ist, wobei die beiden Elektroden (15, 20) einen Kondensator (25) bilden, der entweder als Zylinderkondensator ausgebildet ist, bei dem die zweite Elek-trode (20) die erste Elektrode (15) unter Ausbildung des Zwischenraums (26) umgibt, durch den das Frittieröl zur Messung seiner Kapazität hindurchleitbar ist, oder als Rohrkondensator, bei dem die beiden Elektroden (15, 20) als Halbschalen ausgebildet sind, deren konkave Flächen sich unter Ausbildung eines Zwischenraums (26) gegenüber liegen, durch den das Frittieröl zur Messung seiner Kapazität hindurchleitbar ist,
- mit einem ersten Temperatursensor (35) zur Messung der Temperatur des zu vermessenden Frittieröls, und
- mit einer Auswerteeinheit (50) eingerichtet zur Erfassung der gemessenen Kapazität des Kondensators (25) sowie der gemessenen Temperatur, zur Digitalisierung dieser Messwerte und zur diese Messwerte verwendenden Berechnung der polaren Anteile in dem Frittieröl, wobei diese polaren Anteile ein Kriterium für die Qualität des Frittieröls sind,
**dadurch gekennzeichnet, dass** ein zweiter Temperatursensor (37) vorgesehen ist, und dass die Auswerteeinheit (50) dafür eingerichtet ist, dessen Temperaturmesswerte mit den Temperaturmesswerten des ersten Temperatursensors (35) in eine kombinierte, vorzugsweise mittlere, Temperatur umzurechnen.

2. Ölqualitätssensor nach dem vorhergehenden Anspruch, **dadurch gekennzeichnet, dass** einer der beiden Temperatursensoren (35, 37) in unmittelbarer Nähe der ersten Elektrode (15) und der andere Temperatursensor (37, 35) in unmittelbarer Nähe der zweiten Elektrode (20) angeordnet ist.

3. Ölqualitätssensor nach mindestens einem der vorhergehenden Ansprüche, **dadurch gekennzeichnet, dass** bei Ausbildung des Kondensators (25) als Zylinderkondensator der eine Temperatursensor (35) innerhalb eines Hohlabschnitts (16) der ersten, inneren Elektrode (15) und/oder der andere Temperatursensor (37) auf der der ersten Elektrode (15) abgewandten Außenseite der zweiten, äußeren Elektrode (20) angeordnet ist.

4. Ölqualitätssensor nach mindestens einem der vorhergehenden Ansprüche, **dadurch gekennzeichnet, dass** die Mittelachse(n) (7, 12) der Zulauf- und/oder Ablauföffnung (6, 11) in einem Winkel, vorzugsweise in einem Winkel von 90°, zur Längsachse (27) des Kondensators (25) verläuft bzw. verlaufen.

5. Ölqualitätssensor nach mindestens einem der vorhergehenden Ansprüche, **dadurch gekennzeichnet, dass** an einer Stirnseite des Hohlraums (13) sich rückwärtig eine Kammer (44) anschließt, welche zur Aufnahme der Messelektronik (49), insbesondere der Auswerteeinheit (50), dient, welche mit den zu den beiden Elektroden (15, 20) und dem oder den Temperatursensoren (35, 37) führenden elektrischen Leitungen (17, 21, 36, 38) verbunden sind, wobei außerdem eine digitale Schnittstelle (53) an oder in der Kammer (44) angeordnet ist, welche mit der Messelektronik (49) verbunden ist.

6. Ölqualitätssensor nach mindestens einem der vorhergehenden Ansprüche, **dadurch gekennzeichnet, dass** ein elektronischer Speicher (52) vorgesehen ist, in dem Korrelationsfunktionen hinterlegt sind, welche die Korrelation zwischen den Kapazitätsmesswerten und unterschiedlichen Ölsorten widergeben, und auf welche die Auswerteeinheit (50) je nach ausgewählter Ölsorte zur Berechnung der polaren Anteile aus der gemessenen Kapazität und der Temperatur zugreift.

7. Frittiervorrichtung mit einem Frittierbecken (62) zur Aufnahme von Frittieröl zum Frittieren von Frittiergut, mit einer Frittierwanne (64), die über eine Zuführleitung (73) und eine Abführleitung (74) mit dem Frittierbecken (62) verbunden ist, mit einer Filtereinrichtung (66) zum Filtern des Frittieröls in der Zuführ- oder Abführleitung (73, 74) oder im Bereich des Frittierbeckens (62) oder der Frittierwanne (64), sowie mit einer Pumpe (68) zum Fördern des Frittieröls durch die Zu- und Ablaufleitungen (73, 74) und das Frittierbecken (62) und die Frittierwanne (64), wobei ein Ölqualitätssensor (1) mit zwei Temperatursensoren (35, 37) und einer Auswerteeinheit (50) nach einem der vorhergehenden Ansprüche in eine Frittieröl führende Leitung (73, 74; 75) der Frittiervorrichtung (60) eingebaut ist.

8. Frittiervorrichtung nach dem vorhergehenden Anspruch, **dadurch gekennzeichnet, dass** der Ölqualitätssensor (1) in einen die Filtereinrichtung (66) umfassenden Kreislauf oder in einem eigens für den Ölqualitätssensor (1) vorgesehenen Nebenkreislauf (Messkreislauf) eingebaut ist.

9. Frittiervorrichtung nach Anspruch 7 oder 8, **dadurch gekennzeichnet, dass** der Ölqualitätssensor (1) derart angeordnet ist, dass die Längsachse (27) des Kondensators (25) einen Winkel im Bereich von 20° bis 90°, vorzugsweise größer als 30°, mit der Horizontalen bildet.

10. Frittiervorrichtung nach mindestens einem der Ansprüche 7-9, **dadurch gekennzeichnet, dass** der Ölqualitätssensor (1) und die Auswerteeinheit (50) derart eingerichtet sind, dass ein mehrmaliges Über- oder Unterschreiten eines oder mehrerer vorgegebener Schwellwerte der gemessenen Kapazität in einer vorgegebenen Zeit detektiert und an eine übergeordnete Einrichtung, beispielsweise an die Steuereinrichtung der Frittiervorrichtung (60), gesendet werden kann, welche aus den Messwertschwankungen auf die Anwesenheit von Luft im System schließen kann und die Ausgabe eines Signals, beispielsweise ein Warn- oder Wartungssignal, veranlasst.

11. Frittiervorrichtung nach mindestens einem der Ansprüche 7-10, **dadurch gekennzeichnet, dass** der Ölqualitätssensor (1) und die Auswerteeinheit (50) derart eingerichtet sind, dass ein Unter- oder Überschreiten eines Schwellwerts der gemessenen Kapazität über einen vorgegebenen Zeitraum hinweg detektiert und an eine übergeordnete Einrichtung, beispielsweise an die Steuereinrichtung der Frittiervorrichtung (60), gesendet werden kann, welche aus der Schwellwertunter- oder -überschreitung auf einen unterbrochenen Ölfluss durch den Ölqualitätssensor (1) zu schließen vermag und die Ausgabe eines Signals, beispielsweise eines Warnsignals oder eines Steuersignals zur Abschaltung der Pumpe (68), veranlasst.

## Claims

1. Oil quality sensor to determine the quality of deep-frying oil by measuring the capacitance of the deep-frying oil in a deep fryer:
- with a housing (2) and a hollow space (13) in the housing (2), through which the deep-frying oil can be guided,
- with an inlet opening (6) to introduce deep-frying oil in the hollow space (13),
- with a drain opening (11) to drain the deep-frying oil out of the hollow space (13),
- with a first electrode (15) that extends along the hollow space (13),
- with a second, electrode (20) that extends along the hollow space (13), arranged opposite the first electrode (15), in which case the two electrodes (15, 20) form a capacitor (25) being configured either as a cylinder capacitor, in which the second electrode (20) encloses the first electrode (15) and through which the deep-frying oil can be guided to measure its capacitance, or as a tube capacitor, in which the two electrodes (15, 20) are executed as half shells whose concave surfaces lie opposite each other by forming a space (26) through which the deep-frying oil can be guided to measure its capacitance,
- with a first temperature sensor (35) to measure the temperature of the deep-frying oil that needs to be measured, and
- with an evaluation unit (50) configured to record the measured capacitance of the capacitor (25) and the measured temperature, to digitalize these measured values and to calculate the polar fractions in the deep-frying oil by using these measured values, in which case these polar fractions are a criterion for the quality of the deep-frying oil **characterized in that** a second temperature sensor (37) is provided and that the evaluation unit (50) is configured to convert the measured temperature values of said second temperature sensor (37) together with the measured temperature values of the first temperature sensor (35) into a combined preferably average temperature.

2. Oil quality sensor according to the preceding claim, **characterized in that** one of the two temperature sensors (35, 37) is arranged in direct proximity to the first electrode (15) and the other temperature sensor (37, 35) is arranged in direct proximity to the second electrode (20).

3. Oil quality sensor according to at least one of the preceding claims, **characterized in that** when the capacitor (25) is executed as cylinder capacitor, one of the temperature sensors (35) is arranged inside a hollow section (16) of the first, internal electrode (15) and/or the other temperature sensor (37) is arranged on the external side of the second, external electrode (20) that faces away from the first electrode (15).

4. Oil quality sensor according to at least one of the preceding claims, **characterized in that** the middle axis or axes (7, 12) of the inlet and/or drain opening (6, 11) extends or extend at an angle, preferably at an angle of 90°, to the longitudinal axis (27) of the capacitor (25).

5. Oil quality sensor according to at least one of the preceding claims, **characterized in that** a chamber (44) is attached on the rear, to a front side of the hollow space (13), and the former serves to house the electronic measurement system (49), especially the evaluation unit (50), which are connected to the electrical wires (17, 21, 36, 38) leading to the two electrodes (15, 20) and the temperature sensor(s) (35, 37), in which case a digital interface (53) is additionally arranged on or in the chamber (44), which is connected to the electronic measurement system (49).

6. Oil quality sensor according to at least one of the preceding claims, **characterized in that** an electronic storage unit (52) is provided, in which correlation functions are stored that display the correlation between the measured capacitance values and the different types of oil, and to which the evaluation unit (50) has access depending on the selected type of oil to calculate the polar fractions from the measured capacitance and temperature.

7. Deep fryer with a deep fryer basin (62) to receive deep-frying oil for the deep-frying of foods, with a deep fryer tank (64) that is connected to the deep fryer basin (62) through a supply line (73) and a drain line (74), with a filtering device (66) to filter the deep-frying oil in the supply or drain line (73, 74) or in the area of the deep fryer basin (62) or of the deep fryer tank (64), and with a pump (68) to transport the deep-frying oil through the supply and drain lines (73, 74) and the deep fryer basin (62) and the deep fryer tank (64), in which case an oil quality sensor (1) with two temperature sensor(s) (35, 37) and one evaluation unit (50) is installed in a line (73, 74; 75) carrying deep-frying oil of the deep fryer (60) according to one of the preceding claims.

8. Deep fryer according to the preceding claim, **characterized in that** the oil quality sensor (1) is installed in a cycle that comprises the filtering device (66) or in a secondary cycle (measuring cycle) provided expressly for the oil quality sensor (1).

9. Deep fryer according to claim 7 or 8, **characterized in that** the oil quality sensor (1) is arranged in such a way that the longitudinal axis (27) of the capacitor (25) forms an angle with the horizontal within the range of 20° to 90°, preferably larger than 30°.

10. Deep fryer according to at least one of the claims 7-9, **characterized in that** the oil quality sensor (1) and the evaluation unit (50) are configured in such a way that they can detect how many times one or several specified threshold values of the measured capacitance are exceeded or not reached within a specified time and can be forwarded to a higher-ranking installation, for example, to a control device of the deep fryer (60), which can conclude from the fluctuations in the measured value that there is air in the system and induces the emission of a signal, for example, an alarm or maintenance signal.

11. Deep fryer according to at least one of the claims 7-10, **characterized in that** the oil quality sensor (1) and the evaluation unit (50) are configured in such a way that when they detect that a threshold value of the measured capacitance is not reached or exceeded across a specified time period they can send this to a higher-ranking installation, for example, to the control installation of the deep fryer (60), which is capable of concluding from the threshold value that is exceeded or not reached that the oil flow through the oil quality sensor (1) is interrupted and induces the emission of a signal, for example, a warning signal or control signal, to turn off the pump (68).

## Revendications

1. Détecteur de qualité d'huile pour déterminer la qualité d'une huile de friture par mesure de la capacité de l'huile de friture dans un dispositif de friture :
- avec un boîtier (2) et un espace creux (13) dans le boîtier (2), à travers lequel l'huile de friture peut être acheminée,
- avec un orifice d'admission (6) pour l'injection de l'huile de friture dans l'espace creux (13),
- avec un orifice d'écoulement (11) pour écouler l'huile de friture hors de l'espace creux (13),
- avec une première électrode (15) s'étendant le long de l'espace creux (13),
- avec une seconde électrode (20) s'étendant le long de l'espace creux (13), qui est disposée en face de la première électrode (15), sachant que les deux électrodes (15, 20) forment un condensateur (25), qui se présente sous la forme soit d'un condensateur cylindrique, dans lequel la seconde électrode (20) entoure la première électrode (15) en formant l'espace intermédiaire (26), à travers lequel l'huile de friture peut être acheminée pour la mesure de sa capacité, soit d'un condensateur tubulaire, dans lequel les deux électrodes (15, 20) se présentent sous la forme de semi-coques, dont les surfaces concaves sont opposées l'une à l'autre en formant l'espace intermédiaire (26), à travers lequel l'huile de friture peut être acheminée pour la mesure de sa capacité,
- avec une première sonde de température (35) pour la mesure de la température de l'huile de friture devant être mesurée, et
- avec une unité d'exploitation (50) configurée pour la saisie de la capacité mesurée du condensateur (25) et de la température mesurée, pour la numérisation de ces valeurs de mesure et pour le calcul, sur la base de ces valeurs de mesure, des composés polaires dans l'huile de friture, sachant que ces composés polaires sont un critère pour la qualité de l'huile de friture,
**caractérisé en ce qu'**il est prévu une seconde sonde de température (37), et que l'unité d'exploitation (50) est configurée pour convertir ses valeurs de mesure de température avec les valeurs de mesure de température de la première sonde de température (35) en une température combinée, de préférence en une température moyenne.

2. Détecteur de qualité d'huile selon la revendication précédente, **caractérisé en ce que** l'une des deux sondes de température (35, 37) est disposée à proximité immédiate de la première électrode (15) et l'autre sonde de température (37, 35) à proximité immédiate de la seconde électrode (20).

3. Détecteur de qualité d'huile selon au moins l'une des revendications précédentes, **caractérisé en ce que**, en cas de réalisation du condensateur (25) en tant que condensateur cylindrique, la une sonde de température (35) est disposée à l'intérieur d'une partie creuse (16) de la première électrode (15) intérieure et/ou l'autre sonde de température (37) sur le côté extérieur de la seconde électrode (20) extérieure éloigné de la première électrode (15).

4. Détecteur de qualité d'huile selon au moins l'une des revendications précédentes, **caractérisé en ce que** l'axe central/les axes centraux (7, 12) de l'orifice d'admission et/ou d'écoulèment (6, 11) s'étend/s'étendent à un angle, de préférence à un angle de 90°, avec l'axe longitudinal (27) du condensateur (25).

5. Détecteur de qualité d'huile selon au moins l'une des revendications précédentes, **caractérisé en ce qu'**un compartiment (44) s'enchaîne à l'arrière sur une face frontale de l'espace creux (13), lequel compartiment sert à accueillir l'électronique de mesure (49), en particulier l'unité d'exploitation (50), laquelle est reliée avec les lignes électriques (17, 21, 36, 38) conduisant aux deux électrodes (15, 20) et à la ou aux sondes de température (35, 37), sachant qu'une interface numérique (53) est en outre disposée au ou dans le compartiment (44), laquelle est reliée avec l'électronique de mesure (49).

6. Détecteur de qualité d'huile selon au moins l'une des revendications précédentes, **caractérisé en ce qu'**il est prévu une mémoire électronique (52), dans laquelle sont enregistrées des fonctions de corrélation, lesquelles reproduisent la corrélation entre les valeurs de mesure de capacité et différents types d'huile, et auxquelles accède l'unité d'exploitation (50), selon le type d'huile sélectionné, pour le calcul des composés polaires à partir de la capacité mesurée et de la température.

7. Dispositif de friture avec un bassin de friture (62) pour accueillir une huile de friture pour frire un article à frire, avec une cuve de friture (64), qui est reliée avec le bassin de friture (62) par un conduit d'alimentation (73) et un conduit d'évacuation (74), et avec un dispositif de filtration (66) pour filtrer l'huile de friture dans le conduit d'alimentation ou d'évacuation (73, 74) ou dans la zone du bassin de friture (62) ou de la cuve de friture (64), ainsi qu'avec une pompe (68) pour refouler l'huile de friture à travers les conduits d'alimentation et d'évacuation (73, 74), le bassin de friture (62) et la cuve de friture (64), sachant qu'un détecteur de qualité d'huile (1) avec deux sondes de température (35, 37) et une unité d'exploitation (50) selon l'une des revendications précédentes est incorporé dans un conduit (73, 74 ; 75) d'huile du dispositif de friture (60).

8. Dispositif de friture selon la revendication précédente, **caractérisé en ce que** le détecteur de qualité d'huile (1) est incorporé dans un circuit comprenant le dispositif de filtration (66) ou dans un circuit secondaire (circuit de mesure) prévu spécialement pour le détecteur de qualité d'huile (1).

9. Dispositif de friture selon la revendication 7 ou 8, **caractérisé en ce que** le détecteur de qualité d'huile (1) est disposé de manière à ce que l'axe longitudinal (27) du condensateur (25) forme avec l'horizontale une angle compris dans la plage de 20° à 90°, de préférence supérieur à 30°.

10. Dispositif de friture selon l'une au moins des revendications 7 - 9, **caractérisé en ce que** le détecteur de qualité d'huile (1) et l'unité d'exploitation (50) sont configurés de sorte qu'un écart en plus ou en moins survenant à plusieurs reprises, dans un intervalle de temps prédéterminé, par rapport à une ou plusieurs valeurs seuils prédéterminées de la capacité mesurée, puisse être détecté et transmis à un dispositif supérieur, par exemple à un dispositif de commande du dispositif de friture (60), lequel dispositif de commande est capable, à partir des fluctuations des valeurs de mesure, de déduire de la présence d'air dans le système et de déclencher l'émission d'un signal, par exemple d'un signal d'avertissement ou de maintenance.

11. Dispositif de friture selon l'une au moins des revendications 7 - 10, **caractérisé en ce que** le détecteur de qualité d'huile (1) et l'unité d'exploitation (50) sont configurés de sorte qu'un écart en plus ou en moins par rapport à une valeur seuil prédéterminée de la capacité mesurée, sur un intervalle de temps prédéterminé, puisse être détecté et transmis à un dispositif supérieur, par exemple au dispositif de commande du dispositif de friture (60), lequel dispositif de commande est capable, à partir de l'écart en plus ou en moins par rapport à la valeur seuil, de déduire d'une interruption du flux d'huile à travers le détecteur de qualité d'huile (1) et de déclencher l'émission d'un signal, par exemple d'un signal d'avertissement ou d'un signal de commande pour l'arrêt de la pompe (68).
